# EUROPEAN PATENT APPLICATION

(11) **EP 1 321 464 A1**
(43) Date of publication of application: **25.06.2003**
(21) Application number: 01130756.8
(22) Date of filing: 21.12.2001
(51) Int. Cl.: C07D 307/88, C07D 307/87

(54) **Process for the preparation of 5-cyanophthalide and intermediates useful therein**

(71) Applicant: ICROM S.p.A., 20123 Milano (IT)
(72) Inventor: Leone, Mario, 20067 Paullo (MI) (IT)
(74) Representative: Mancini, Vincenzo, Dr.

(57) **Abstract**

A process for the preparation of 5-cyanophtalide is disclosed which comprises reacting a pharmaceutically acceptable salt of 5-carboxyphtalide with an alkyl chloroformate so to obtain a mixed anhydride, the latter being reacted with an alkyldisilazane so to obtain an amide, the latter being reacted with a chloride so to obtain substantially pure 5-cyanophtalide. The pharmaceutically acceptable salts of 5-carboxyphtalide are useful intermediates in the process of the invention which can be carried out as a one-pot procedure.

## Description

The present invention relates to a process for the preparation of 5-cyanophtalide and intermediates useful therein.

5-Cyanophtalide is a key intermediate in the preparation of citalopram, a selective serotonin reuptake inhibitor (*Acta Psychiatr.Scand. 478*, *75(1987))* used, as an antidepressant, in geriatrics and in the cure of obesity and alcoholism, as well.

Most of the syntheses of 5-cyanophtalide (III) comprise converting 5-carboxyphtalide (II) according to the following reaction scheme:

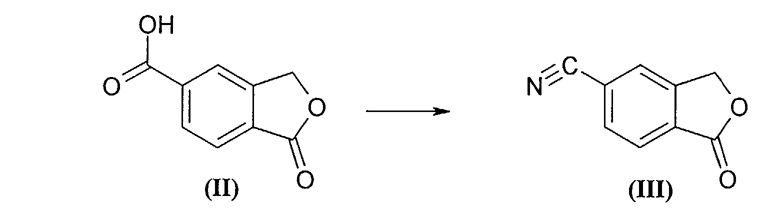

Alkaline or ammonium salts of carboxylic acids are known to be readily soluble in water; accordingly, 5-carboxyphtalide is currently crystallised by precipitating it in the acid form.

US 3,607,884 discloses preparing 5-carboxyphtalide by reacting terephtalic acid in liquid SO₃ with formaldeide, in 1:1 molar ratio, at about 120-180°C. 5-Carboxyphtalide is described to dissolve in a basic solution as a difunctional acid whereas the corresponding hydrate, 2-hydroxymethylterephtalic acid, is isolated from basic solution by adjusting pH.

Also WO 01/32642 and WO 01/32643 describe preparing 5-carboxyphtalide starting from terephtalic acid and reacting it with either paraformaldheyde in oleum or with paraformaldheyde or trioxane in the presence of a Lewis or a mineral acid. Both documents disclose that 5-carboxyphtalide may be dissolved in aqueous medium by adjusting pH to about 6.7 to 7.3 and that it can be precipitated by acidification; further, despite the use of sodium hydroxide, no crystallization of the sodium salt of 5-carboxyphtalide is disclosed.

Yet, terephtalic acid is known to be poorly soluble in water as well as in most of the organic solvents, so it is very hard to purify 5-carboxyphtalide from terephtalic acid.

The main impurities deriving from the synthesis of 5-carboxyphtalide, besides terephtalic acid (VIII), are diphtalide derivatives of formula (VI) and (VII):

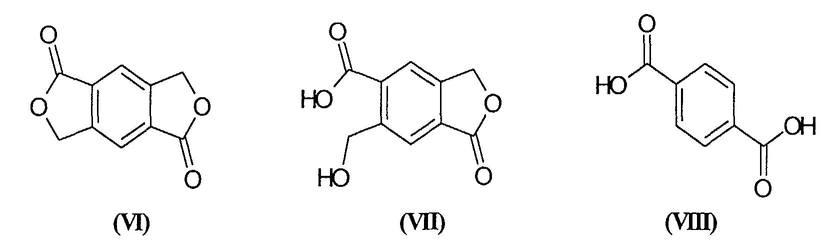

While the derivative of formula (VI) is not soluble in neutral or weak alkaline medium, the derivative of formula (VII) and terephtalic acid are soluble just like 5-carboxyphtalide which, accordingly, results to be contaminated; further, the derivatives of formulae (VII) and (VIII) can react just as 5-carboxyphtalide and originate nitrile-type impurities.

GB 1,578,989 describes a synthesis of 5-carboxyphtalide comprising electrolytically reducing trimellitic anhydride in an aqueous solution, preferably of ammonium carbonate and ammonium hydroxide, at a temperature below 100°C. The synthesis comes out to be quite inadequate since a very low purity is obtained, this requiring further steps and causing therefore higher costs; no crystallization of the ammonium salt of 5-carboxyphtalide is disclosed.

The conversion of a carboxylic acid into the corresponding nitrile is a well known reaction (see, f.i., Larock in *"Comprehensive organic transformation"* II ed., Wiley-VCH).

Nitrile derivatives can be obtained by reacting, for instance, a carboxylic acid with ammonia or urea at high temperatures [*Org. Syn. Coll.* vol. 4, 62(1963); *Syn.* 142(1983); *Org. Syn. Coll.* vol. 3 768(1955)] yet, the reaction can be carried out on heat-resistant substrates only.

Another synthesis of nitrile derivatives involves reacting a carboxylic acid with chlorosulfonylisocyanate and dimethylformamide (DMF) or triethylamine [*Syn. Commun.* 12, 25(1982)); *Org. Syn. Coll.* vol. 6 304(1988); *T.L.* 1631(1968)] which results to be, however, dangerous, toxic and expensive.

Still another alternative synthesis comprises reacting a carboxylic acid with cyanogen bromide at high temperature *[Can. J. Chem.* 1127, 31(1953); *Can. J. Chem.* 1256, 36(1958)] yet, this coming out to be highly poisoning, toxic and suitable for heat-resistant substrates only.

A further possibility comprises reacting a carboxylic acid -and using pyridine as a solvent-with methanesulfonylchloride and ammonia *[J. Org. Prep. Proc. Int.* 396, 14(1982)], this being a quite good procedure only on a lab-scale because pyridine is an evident limit to scale up the process.

WO 00/39112 discloses the synthesis of 5-cyanophtalide via a two-step procedure, wherein 5-carboxyphtalide is first transformed into a secondary amine, preferably via an acid chloride or an ester, and then dehydrated, preferably with ammonia or a C₁₋₆ primary amine. While the acid chloride is highly reactive yet, like all chlorides, very irritant, this making the isolation and handling of the solid quite dangerous and hazardous on an industrial scale, the ester, on the other hand, is not as reactive as the acid chloride and requires long reaction time and high temperature.

WO 00/44738 discloses a one pot synthesis of 5-cyanophtalide, the method comprising reacting 5-carboxyphtalide with a dehydrating agent and a sulphonamide, preferably using sulfolane or acetonitrile as solvents, at 135°C. The reaction provides the formation of the correspondent haloformyl derivative, optionally isolated, then converted into the amide derivative thereof, which decomposes at high temperature.

The low purity of 5-carboxyphtalide obtained by the known methods evidently reflects on the purity of 5-cyanophtalide and, consequently, on that one of citalopram. Therefore, there is still the need for a cost-effective industrial process for the preparation of substantially pure 5-cyanophtalide.

According to a first aspect, the present invention concerns a process for the preparation of 5-cyanophtalide comprising:
(a) reacting a pharmaceutically acceptable salt of 5-carboxyphtalide of formula (I), wherein M is a pharmaceutically acceptable cation, with an alkyl chloroformate of formula R'OCOCl, wherein R' is a C₂₋ ₄, linear or branched, alkyl, so to obtain a mixed anhydride of formula (IV), according to the following reaction scheme: 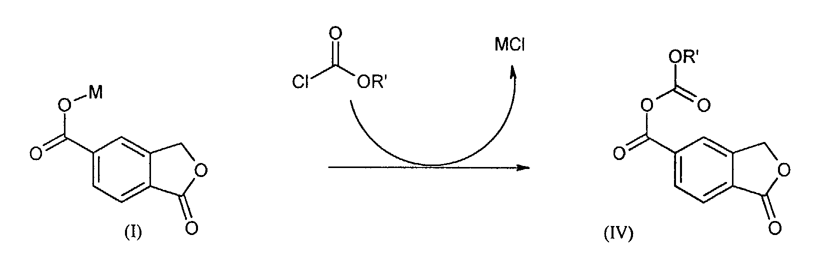
(b) reacting the mixed anhydride with an alkyldisilazane of formula [(R)₃Si]₂NH, wherein R is independently H or a C₁₋₄, linear or branched, alkyl, so to obtain an amide of formula (V), according to the following reaction scheme: 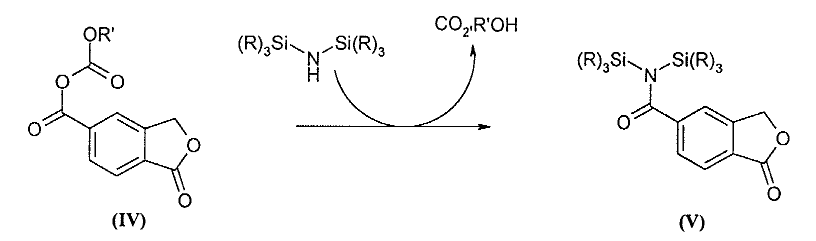
(c) reacting the amide with a water-hydrolysable chloride so to obtain 5-cyanophtalide of formula (III), according to the following reaction scheme:

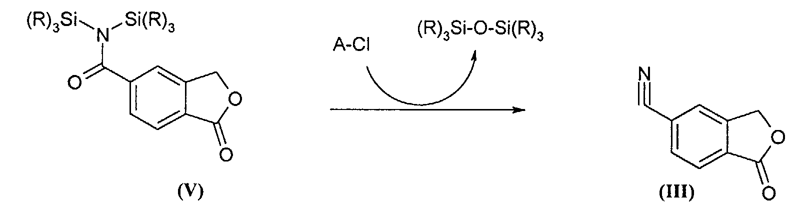

In the present specification, the expression "water-hydrolysable chloride" is meant to indicate a chloride which is hydrolysed in water, therefore forming hydrochloric acid. Preferably, the water-hydrolysable chloride has the formula A-Cl, wherein A is an inorganic group selected from SOCl₂, POCl₂, PCl₄, or an organic group selected from CH₃CO, CF₃CO, CH₃SO₂, CF₃SO₂ or (CH₃)₂N⁺=C, the latter being known as Vilsmeier reagent.

Surprisingly, no relevant side reactions occur carrying out the process of the invention; the high purity of the compounds of formula (I), as starting materials, reflects on the high purity of 5-cyanophtalide which is obtained in high yields as well.

In fact, it has been found that 5-carboxyphtalide can be precipitated as a pharmaceutical acceptable salt, even from water, in a substantially pure form, with no or very low content of humidity. This crystallization is indeed very selective and leaves the unreacted terephtalic acid (VIII), which is very insoluble in water as free acid and yet readily soluble as a salt, together with the salified by-product of formula (VII) in the mother liquor.

The present process results therefore to be a cost-effective industrial process for the preparation of substantially pure (i.e. a purity equal or greater than 99%) 5-cyanophtalide.

Preferably, the process can be performed as a one-pot procedure in a suitable organic, aprotic, anhydrous solvent, easily selected by the skilled man according to the common knowledge of the field; preferred solvents can be selected, for instance among chlorinated hydrocarbons, e.g. dichloromethane, chloroform; aromatic hydrocarbons, e.g. toluene, xylene; amides, e.g. dimethylformamide, dimethylacetamide; ethers, e.g. tetrahydrofurane; or a mixture thereof. Amides are the most preferred solvents.

The pharmaceutically acceptable cation M for the compounds of formula (I) is preferably selected from an alkaline metal, ammonium or a primary, secondary, or tertiary alkyl-amine; most preferred are sodium, ammonium or triethylammonium.

The C₂₋₄, linear or branched, alkyl chloroformate is preferably ethylchloroformate, isopropylchloroformate or isobutylchloroformate, in an amount preferably ranging from the stoichiometric one to an excess of 10 up to 40% with respect to the compounds of formula (I).

The concentration of the compounds of formula (I) in step (a) is not a critical aspect yet, a concentration between 50 and 200 g/l is preferred.

Also temperature, in step (a), is not critical yet, it preferably ranges between -40°C and 30°C, most preferably between -10°C and 10°C.

Once the mixed anhydride of formula (IV) is obtained, step (b) is performed; R, in the alkyldisilazane of formula [(R)₃Si]₂NH, is preferably and independently methyl or ethyl; most preferred alkyldisilazanes are hexamethyldisilazane and tetramethyldisilazane whereas its amount ranges preferably from the stoichiometric one to an excess of 10 up to 100% with respect to the compounds of formula (IV).

Temperature, in step (b), preferably ranges between -10°C and 30°C, most preferably between 10°C and 30°C.

Temperature, in step (c), preferably ranges between 0°C and 150°C, most preferably between 10°C and 60°C, whereas the amount of the chloride ranges preferably between 1 and 10 molar equivalents with respect to the compounds of formula (V); most preferred chlorides are thionylchloride, phosphorous oxychloride, phosphorous chloride, phosphorous pentachloride, mesylchloride, acetylchloride, trifluoroacetylchloride, triflic chloride, (chloromethylene)dimethyliminium chloride.

The Applicant also realised that even if in some of the known syntheses of 5-carboxyphtalide, its salification could have occurred *in situ*, never the isolation of any salt was disclosed.

According to a second aspect, the invention concerns a compound of formula (I): 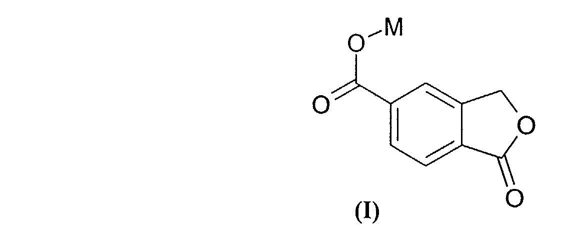 wherein M is as above defined.

The compounds of formula (I), as well as 5-carboxyphtalide (CAS number 4792-29-4), can be prepared according to techniques commonly known in the field; for instance, they can be prepared directly and *in situ,* in a suitable organic solvent, from 5-carboxyphtalide made, in its turn and for instance, from terephtalic acid and a formaldehyde source in liquid SO₃, according to the process disclosed in US 3,607,884 which is herein incorporated as far as the synthesis of 5-carboxyphtalide is concerned, and then finally reacted in order to form 5-cyanophtalide.

The following examples illustrate the invention without limiting it.

All the products were analysed by HPLC using a column RP18, 250x4 mm, the eluent being 25% acetonitrile in 0.001M H₃PO₄; detector wavelength: 240 nm. All analyses were normalised.

### Example 1: 5-carboxyphtalide sodium salt

30 g of crude 5-carboxyphtalide (2% of terephtalic acid, 8% of diphtalide), prepared according to US 3,607,884, were dissolved in 150 ml of water with 15 g of sodium bicarbonate.

The resulting solution was warmed at 80°C and 3 g of activated carbon (DARCO®) were added. The solution was then filtered, washing the filter with hot water and then adding 20 g of sodium chloride, subsequently cooling at room temperature.

The precipitate was harvested and washed with cold water and isopropanol; after drying, 25 g of a pinkish product were obtained (0.1% of terephtalic acid, no diphtalide, K.F.: 3%). The product showed no melting point until 300°C.

IR (KBr): broad 3500-3100 cm⁻¹ O-H stretch (water); 3068 cm⁻¹ C-H stretch (aromatic ring); 2984 cm⁻¹ C-H stretch (aliphatic); 1764 cm⁻¹ C=O stretch (lactonic ring); 1625cm⁻¹, 1609cm⁻¹ C=C stretch (aromatic ring); 1580cm⁻¹ C=O asymmetric stretch, 1396 cm⁻¹ C=O symmetric stretch (carboxylate ion).

### Example 2: 5-carboxyphtalide ammonium salt

30 g of crude 5-carboxyphtalide (2% of terephtalic acid, 8% of diphtalide) were dissolved in 100 ml of water with 12 ml of concentrated ammonium hydroxide.

The solution was warmed at 50°C and then filtered; after the filtration, it was cooled and allowed to stand overnight at 5°C.

The precipitate was harvested and washed with cold water and isopropanol; after drying, 18 g of an off-white product were obtained (neither terephtalic acid nor diphtalide, K.F.: 0%). The product showed slow decomposition starting at about 280°C.

IR (KBr): 3254 cm⁻¹ C=O stretch overtone band (lactonic ring); broad 3100-2600 cm⁻¹ N-H stretch (ammonium ion); 1744 cm⁻¹ C=O stretch (lactonic ring); 1625cm⁻¹, 1609cm⁻¹ C=C stretch (aromatic ring); 1561cm⁻¹ C=O asymmetric stretch, 1378 cm⁻¹ C=O symmetric stretch (carboxylate ion).

### Example 3: 5-carboxyphtalide triethylammonium salt

The procedure of example 1 was repeated, yet the wet sodium salt was dissolved in 200 ml of water at 80°C, decoloured with activated carbon and filtered.

After the filtration, the solution was maintained at 80°C and 15% sulphuric acid was slowly added until pH 1 was reached; the product, crystallized as free acid, was harvested and dried under vacuum.

20 g of an almost white solid product were obtained. The acid, substantially pure (m.p. 293°C, literature: 285-290°C), was suspended in 100 ml of anhydrous dimethylacetamide; 15.5 ml of thrietylamine were slowly added thereto thus dissolving entirely the solid product and then titrating the solution with 0.1N perchloric acid (solution in acetic acid). The solution of the title product showed a potency of 26% (weight/volume). This *in situ* salt solution was directly used for the next step.

### Example 4: 5-cyanophtalide

The solution obtained in example 3, having a potency of 20 g expressed like 5-carboxyphtalide, was cooled under nitrogen to -10°C, then adding 13 ml of ethylchloroformate; the reaction was stirred at 0°C for 1 hour then cooled again at -10°C, adding 28 ml of hexamethyldisilazane and letting the temperature rise up to 25°C.

The reaction was stirred to room temperature for 2 hours, then 20 ml of thionyl chloride were dropped in, finally warming the solution up to 140°C for 6 hours.

The resulting solution was cooled and poured into water, harvesting the solid so obtained and washing the latter with water. After drying, 15 g of the product were obtained: m.p. 198°C (literature: 202-203°C). This crude product can be recrystallised from toluene in order to obtain a white solid with a melting point of 202°C and a purity (HPLC) of 100%.

IR (KBr): 3497 cm⁻¹ C=O stretch overtone band (lactonic ring); 3113 cm⁻¹, 3094 C-H stretch (aromatic ring); 2962 cm⁻¹ C-H stretch (aliphatic); 2232 cm⁻¹ C≡N stretch; 1759 cm⁻¹ C=O stretch (lactonic ring); 1625cm⁻¹, 1609cm⁻¹ C=C stretch (aromatic ring); 1561cm⁻¹ C=O asymmetric stretch, 1378 cm⁻¹ C=O symmetric stretch (carboxylate ion).

### Example 5: 5-cyanophtalide

The same procedure of example 4 was repeated yet using dimethylformamide as a solvent.

After recrystallization from toluene, 12 g of product were obtained as a pale grey solid with a melting point of 200°C and a purity of 99.5%.

The IR spectrum showed the same wavelengths reported as to example 4.

### Example 6: 5-cyanophtalide

The procedure of example 5 was repeated using 22 g of 5-carboxyphtalide ammonium salt (20 g of potency).

14 g of the crude product were isolated by adding water to the reaction pot; the melting point resulted to be 202°C whereas purity was 99.8%.

The IR spectrum showed the same wavelengths reported as to example 4.

## Claims

1. Process for the preparation of 5-cyanophtalide comprising:
(a) reacting a pharmaceutically acceptable salt of 5-carboxyphtalide of formula (I), wherein M is a pharmaceutically acceptable cation, with an alkyl chloroformate of formula R'OCOCl, wherein R' is a C₂₋ ₄, linear or branched, alkyl, so to obtain a mixed anhydride of formula (IV), according to the following reaction scheme: 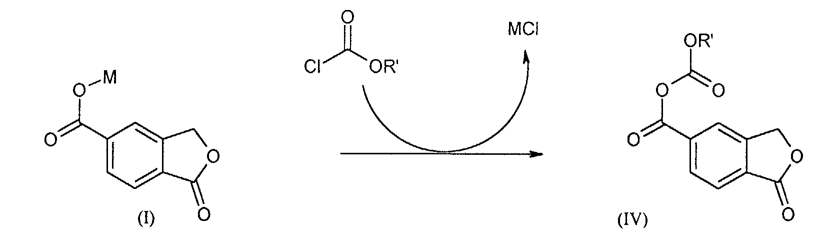
(b) reacting the mixed anhydride with an alkyldisilazane of formula [(R)₃Si]₂NH, wherein R is independently H or a C₁₋₄, linear or branched, alkyl, so to obtain an amide of formula (V), according to the following reaction scheme: 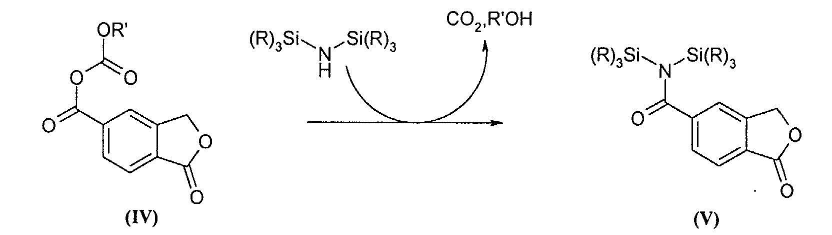
(c) reacting the amide with a water-hydrolysable chloride so to obtain 5-cyanophtalide of formula (III), according to the following reaction scheme:

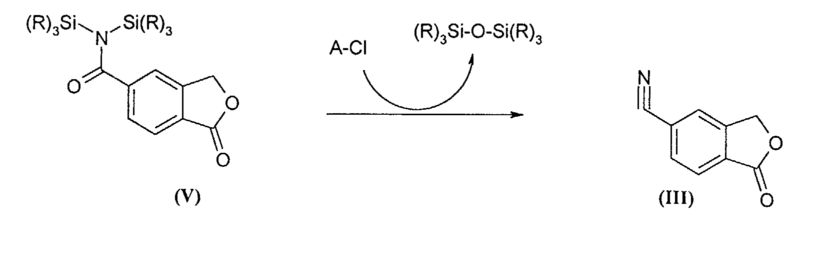

2. A process according to claim 1, wherein M is selected from an alkaline metal, ammonium or a primary, secondary, or tertiary alkyl-amine.

3. A process according to claim 1 or 2, wherein M is sodium, ammonium or triethylammonium.

4. A process according to any of the previous claims, wherein R' is ethyl, isopropyl or isobutyl.

5. A process according to any of the previous claims, wherein the alkyl chloroformate is in an amount ranging from the stoichiometric one to an excess of 10 up to 40% with respect to the compounds of formula (I).

6. A process according to any of the previous claims, wherein the amount of the compound of formula (I) ranges between 50 and 200 g/l.

7. A process according to any of the previous claims, wherein temperature, in step (a), ranges between -40°C and 30°C.

8. A process according to any of the previous claims, wherein temperature, in step (a), ranges between -10°C and 10°C.

9. A process according to any of the previous claims, wherein R is independently methyl or ethyl.

10. A process according to any of the previous claims, wherein the alkyldisilazane is hexamethyldisilazane or tetramethyldisilazane.

11. A process according to any of the previous claims, wherein the amount of the alkyldisilazane ranges between the stoichiometric one and an excess of 10 up to 100% with respect to the compounds of formula (IV).

12. A process according to any of the previous claims, wherein temperature, in step (b), ranges between -10°C and 30°C.

13. A process according to any of the previous claims, wherein temperature, in step (b), ranges between 10°C and 30°C.

14. A process according to any of the previous claims, wherein the chloride has the formula A-Cl, wherein A is an inorganic group selected from SOCl₂, POCl₂, PCl₄, or an organic group selected from CH₃CO, CF₃CO, CH₃SO₂, CF₃SO₂ or (CH₃)₂N⁺=C.

15. A process according to any of the previous claims, wherein the chloride is selected from thionylchloride, phosphorous oxychloride, phosphorous chloride, phosphorous pentachloride, mesylchloride, acetylchloride, trifluoroacetylchloride, triflic chloride, (chloromethylene)dimethyliminium chloride.

16. A process according to any of the previous claims, wherein the amount of the chloride ranges between 1 and 10 molar equivalents with respect to the compounds of formula (V).

17. A process according to any of the previous claims, wherein temperature, in step (c), ranges between 0°C and 150°C,

18. A process according to any of the previous claims, wherein temperature, in step (c), ranges between 10°C and 60°C.

19. A compound of formula (I): 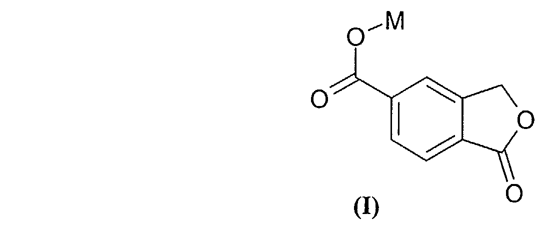 wherein M is an alkali metal, ammonium or a primary, secondary or tertiary amine.

20. A compound according to claim 1 wherein M is sodium.

21. A compound according to claim 1 wherein M is ammonium.

22. A compound according to claim 1 wherein M is triethylammonium.
